# EUROPÄISCHE PATENTSCHRIFT

(11) **EP 2 623 215 B1**
(45) Veröffentlichungstag und Bekanntmachung des Hinweises auf die Patenterteilung: **26.03.2014**
(21) Anmeldenummer: 12000648.1
(22) Anmeldetag: 01.02.2012
(51) Int. Cl.: B05D 7/00, B05D 3/14, B05D 5/04, B05D 1/00, B05D 3/04

(54) **Hydrophilierende Plasmabeschichtung**
Hydrophilic plasma coating
Revêtement par plasma hydrophile

(43) Veröffentlichungstag der Anmeldung: 07.08.2013
(73) Patentinhaber: Bioenergy Capital AG, 50668 Köln (DE)
(72) Erfinder: Görne, Martin, 22337 Hamburg (DE); Kordick, Thomas, 63773 Goldbach (DE)
(74) Vertreter: Diehl & Partner GbR

(56) Entgegenhaltungen:
- EP-A2- 0 896 035
- US-A- 5 080 924
- US-A- 6 005 160
- US-A1- 2009 069 790

## Beschreibung

Die vorliegende Erfindung betrifft die Oberflächenbehandlung von Werkstücken auf Basis von Biomaterialien und bezieht sich im Besonderen auf ein dauerhaftes Hydrophilieren von Oberflächen solcher Werkstücke mittels plasmaunterstützter chemischer Gasphasenabscheidung (PECVD) und nachfolgender chemischer Gasphasenabscheidung (CVD).

An die biologische Verträglichkeit von Werkstücken, die zum vorübergehenden oder dauerhaften Einsatz an Organen von Menschen oder Tieren vorgesehen sind, wie beispielsweise Kontaktlinsen oder Implantate, werden, um Entzündungsprozesse zu vermeiden, hohe Anforderungen gestellt. Um eine entsprechend hohe Biokompatibilität sicherzustellen, werden zur Herstellung solcher Werkstücke Materialien verwendet, deren Eigenschaften sie sowohl für den jeweiligen Einsatzzweck als auch den damit verbundenen Gewebekontakt prädestinieren.

Die auch als Biokompatibilität bezeichnete biologische Verträglichkeit von Materialien wird zu einem wesentlichen Teil durch deren Oberflächeneigenschaften beeinflusst. Bei Kontaktlinsen ist eine hydrophile Oberfläche entscheidend für eine gute Biokompatibilität. Bei Implantaten im Rahmen von Tissue Engineering (Aufbau von körpereigenem Gewebe) begünstigt eine hydrophile Oberfläche polymerer Gerüstsubstanzen deren Besiedelung durch Gewebezellen und damit den therapeutischen Erfolg. Auch bei *in vitro* Testverfahren mit vitalen Zellen ist eine hydrophile Oberfläche des polymeren Substrats vorteilhaft, um die Zellen zu fixieren.

Ein biokompatibles Hydrophilieren von Oberflächen polymerer Biomaterialien kann, wie z. B. in der internationalen Anmeldung WO 99/57177 beschrieben ist, durch ein Modifizieren der Polymeroberfläche mittels Plasmaoxidation erzielt werden. Es hat sich jedoch gezeigt, dass solcherart hydrophilierte Oberflächen nicht in ausreichendem Maß langzeitstabil sind.

Eine dauerhaftere Hydrophilierung polymerer Biomaterialoberflächen wird durch Beschichten dieser mit einem hydrophilen, biokompatiblen Werkstoff erreicht. Zum Herstellen hydrophiler Oberflächen an Kontaktlinsen aus Polymethylmethacrylat (PMMA) wird in der Patentschrift US 5,080,924 beispielsweise ein Plasmaabscheideprozess zum Aufpolymerisieren der Oberflächen mit Polyacrylsäure vorgeschlagen. Die aufpolymerisierten PMMA-Oberflächen zeigen Benetzungswinkel für Wasser aus dem Bereich von 35 bis 50 Grad und sind für eine ausreichende Benetzung der Materialoberflächen zu groß. Zur weiteren Verringerung des Benetzungswinkels muss die Beschichtung weiterbehandelt werden, beispielsweise durch Aufbringen eines von Acrylsäure verschiedenen weiteren biokompatiblen Materials, das sich mit der Polyacrylsäure vernetzt. Ein solcher mehrere Materialschichten umfassender Prozess erfordert jedoch einen höheren apparativen Aufwand und resultiert auch in längeren Beschichtungszeiten.

Ein Verfahren zum hydrophilieren von Kontaktlinsen ist beispielsweise aus der Anmeldung EP 0 896 035 A2 bekannt. Hydrophil beschichtete Polymer-Werkstücke werden in der US2009/0069790 A1 beschrieben.

Ausgehend vom Dargelegten ist es daher wünschenswert, ein weniger komplexes biokompatibles Beschichten polymerer Biomaterialien anzugeben, das eine langzeitstabile Oberflächenhydrophilierung mit Wasserbenetzungswinkeln von 15 Grad und darunter ermöglicht.

Ein solches Beschichten umfasst ein Verfahren zum Hydrophilieren von Oberflächen polymerer Werkstücke, wobei das Verfahren einen Schritt (a) umfasst zum Reinigen und Aktivieren der Werkstückoberflächen im Rahmen einer Vorbehandlung mit einem auf Grundlage eines Inertgases gebildeten Hochfrequenzgasplasmas, einen Schritt (b) zum Vorbeschichten der vorbehandelten Werkstückoberflächen mit Polyacrylsäure unter Verwendung eines aus einer Gasmischung erzeugten Hochfrequenzgasplasmas, wobei sich die Gasmischung aus einem Inertgas und einem aus biokompatiblen, polymerisierbaren carboxygruppenhaltigen Monomeren gebildeten ersten Gas zusammensetzt, und einen Schritt (c) zum Folgebeschichten der vorbeschichteten Werkstückoberflächen unter Verwendung eines von im Wesentlichen Acrylsäuremonomere enthaltenden zweiten Gases.

Das Beschichten umfasst ferner das Bereitstellen eines polymeres Werkstücks mit einer hydrophilierenden Oberflächenbeschichtung aus Polyacrylsäure, das nach einem die vorgehend spezifizierten Schritte umfassenden Verfahren erhältlich ist, wobei der Benetzungswinkel von Wasser an der mit Polyacrylsäure beschichteten Werkstückoberfläche einen Wert aus dem Bereich von 2 bis weniger als 10 Grad aufweist.

Die mit dem spezifizierten Verfahren beschichteten Werkstücke weisen eine dauerhaft hydrophile Oberfläche ausgezeichneter Benetzungsfähigkeit auf, die bei Kontakt mit Körpergewebe in einer guten Bioverträglichkeit resultiert, wodurch Reizungen des Auges bei entsprechend beschichteten Kontaktlinsen weniger häufig auftreten und sich Körperzellen bei entsprechend beschichteten Gerüstsubstanzen für Tissue Engineering leicht anheften.

Falls es sich nicht deutlich anders aus dem Zusammenhang ergibt, sind in der Beschreibung und den Ansprüchen die Wörter "aufweisen", "umfassend", "beinhalten", "enthalten" "mit" und dergleichen, sowie deren grammatikalischen Abwandlungen, als umfassend im Gegensatz zu einer ausschließlichen oder erschöpfenden Bedeutung aufzufassen; das bedeutet im Sinne von "einschließlich, aber nicht darauf beschränkt".

Bei vorteilhaften Ausführungsformen des Verfahren sind die das erste Gas bildenden biokompatiblen, polymerisierbaren carboxygruppenhaltigen Monomere ausgewählt sind unter (Meth)Acrylsäure und (Meth)Acrylsäureanhydrid, wodurch im Hochfrequenzplasma ein hoher Anteil von Acrylsäuremonomeren erzeugt wird, die sich an der in Schritt (a) des Verfahrens aktivierten Werkstückoberfläche unter Ausbildung kovalenter Bindungen anlagern.

Bei weiter vorteilhaften Ausführungsformen enthält das in Schritt (a) des Verfahrens zur Ausbildung des Hochfrequenzplasmas verwendete Gas das erste Gas in einer Menge, die einem Partialdruck von weniger als einem Zehntel des Partialdrucks des Inertgases entspricht, so dass eine effektive Reinigung und Aktivierung der Werkstückoberfläche sichergestellt ist.

Um eine stabile Anlagerung von Acrylsäuremonomeren an die Werkstückoberfläche zu erhalten wird bei bevorzugten Ausführungsformen in Schritt (b) eine Gasmischung verwendet, bei der der Partialdruck des ersten Gases mindestens ein Viertel und maximal das Doppelte des Partialdrucks des Inertgases beträgt.

Im Hinblick auf den Erhalt einer dichten und stabilen Polyacrylsäurebeschichtung beträgt der Partialdruck des Inertgases am in Schritt (c) verwendeten zweiten Gas bei Ausführungsformen vorzugsweise weniger als ein Zehntel des Partialdrucks des von Acrylsäuremonomeren gebildeten Gases.

Bei bevorzugten Ausführungsformen wird Argon als Inertgas verwendet.

Zur effektiven Kontrolle des Vorbeschichtungsprozesses wird bei Ausführungsformen die in Schritt (b) aufgebrachte Beschichtung mithilfe einer Schichtdickenkontrolleinrichtung überwacht und bei Erreichen eines aus dem Bereich von 50 bis 400 Å ausgewählten Schichtdickenwerts beendet.

Bei besonders bevorzugten Ausführungsformen, bei denen Benetzungswinkel aus dem Bereich von 2 bis weniger als 10 Grad erreicht werden, weist der Druck des Inertgases für das Hochfrequenzplasma in Schritt (a) einen Wert aus dem Bereich von 15 bis 60 mTorr (ca. 2 bis 8 Pa) und der Druck des ersten Gases für das Hochfrequenzplasma in Schritt (b) einen Wert aus dem Bereich von 30 bis 90 mTorr (ca. 4 bis 12 Pa) auf.

Zur Fixierung der Acrylsäurepolymerbeschichtung auf den Werkstückoberflächen umfasst das spezifizierte Verfahren ferner einen Schritt (cb), der unmittelbar an Schritt (b) anschließend ein Drosseln der Inertgaszufuhr und ein Zuführen des zweiten Gases umfasst, wobei der Druck des zweiten Gases in Schritt (c) weniger als 0,3 mTorr (ca. 40 mPa) beträgt.

Zur Begünstigung einer Anlagerung und Vernetzung von Acrylsäuremonomeren an der vorbeschichteten Werkstückoberfläche weisen Ausführungsformen ferner einen Schritt (bc) auf, der unmittel an Schritt (b) oder, wenn ausgeführt an Schritt (cb) anschließend ein Ausschalten des Hochfrequenzplasmas, ein Unterbrechen der Inertgaszufuhr und ein Zuführen des zweiten Gases umfasst, wobei der Druck des zweiten Gases in Schritt (c) zwischen 1,5 und 6 Torr (ca. 0,13 bis 0,8 kPa) beträgt.

Zur Verbesserung der Bioverträglichkeit weisen Ausführungsformen ferner einen an Schritt (c) anschließenden weiteren Schritt (d) zum Entfernen wasserlöslicher Bestandteile aus der Hydrophilierungsschicht mittels Spülen des beschichteten Werkstücks in einem hydrophilen Lösungsmittel wie z. B. in einer isotonischen Kochsalzlösung oder, je nach Verwendungszweck des Werkstücks, in demineralisiertem Wasser auf.

Bei weiterhin bevorzugten Ausführungsformen weist das Werkstück zumindest an seiner Oberfläche ein Material auf, das überwiegend oder im Wesentlichen aus einem Silicon, insbesondere Poly(dimethylsiloxan), einem Siliconhydrogel oder einem porösen bioresorbierbaren Polymer wie PLA oder PLGA gebildet ist. Die Dickenbereiche der Werkstücke betragen bei Ausführungsformen, die auf den ersteren für Kontaktlinsen relevanten Fall bezogen sind, vorzugsweise zwischen 50 und 300 µm, zwischen 5 und 40 µm, oder zwischen 2 und 12 µm. Die Dicke der Beschichtung liegt bei Ausführungsformen mit porösem PLA oder PLGA vorzugsweise zwischen 5 und 40 nm.

Bei Ausführungsformen handelt es sich bei den Werkstücken um Silikon-Kontaktlinsen. Die mit dem Verfahren hergestellte hydrophilierende Oberflächenbeschichtung dieser Werkstücke wird von einer PAA-Schicht mit einer durchschnittlichen Dicke von 5 bis 40 µm gebildet.

Bei anderen Ausführungsformen handelt es sich bei den Werkstücken um eine poröse Matrix aus Poly(α-hydroxycarbonsäuren). Die mit dem Verfahren hergestellte hydrophilierende Oberflächenbeschichtung dieser Werkstücke wird von einer PAA-Schicht mit einer durchschnittlichen Dicke von 5 bis 40 nm gebildet.

Weitere Merkmale der Erfindung ergeben sich aus der nachfolgenden Beschreibung von Ausführungsbeispielen in Verbindung mit den Ansprüchen sowie den Figuren. Die Erfindung ist nicht auf die beschriebenen Ausführungsbeispiele beschränkt, sondern durch den Umfang der beiliegenden Patentansprüche bestimmt. Insbesondere können die einzelnen Merkmale bei erfindungsgemäßen Ausführungsformen in anderer Anzahl und Kombination als bei den untenstehend angeführten Beispielen verwirklicht sein. Bei der nachfolgenden Erläuterung von Ausführungsbeispielen wird auf die beiliegenden Figuren Bezug genommen, von denen
- Figur 1: eine schematische Darstellung zur Veranschaulichung eines Systems für eine biokompatible Beschichtung von polymeren Biomaterialien zeigt,
- Figur 2: ein Flussdiagramm zur Veranschaulichung der für eine Beschichtung polymerer Biomaterialien mit Polyacrylsäure wesentlichen Verfahrensschritte zeigt, und
- Figur 3: ein Fluoreszenz-Diagramm zur Veranschaulichung der mit dem Verfahren nach Figur 2 erreichten Schichtdicke zeigt.

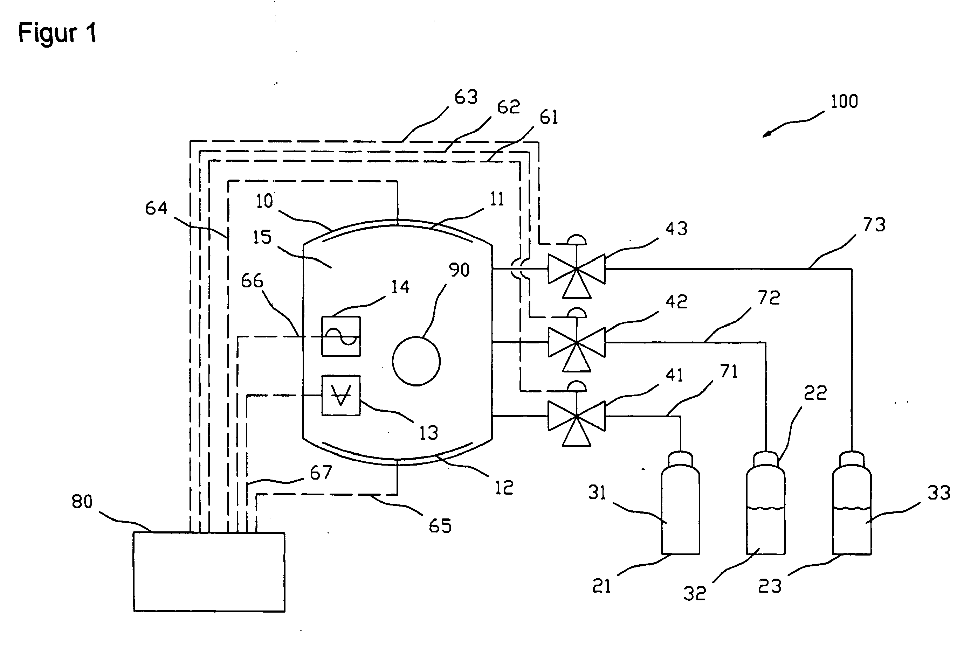

Das in Figur 1 dargestellte Schema veranschaulicht die wesentlichen Komponenten einer Vorrichtung 100 zur Beschichtung polymerer Werkstücke 90 mit einem deren Oberfläche hydrophil ausbildenden Material. Bei den Werkstücken handelt es sich vorzugsweise entweder um Kontaktlinsen und hierbei vorzugsweise um solche aus einem Silicon oder einem Siliconhydrogel, oder um eine für Tissue Engineering geeignete, vorzugsweise aus PLA (Polylactid) oder PLGA (Polylactid-co-Glycolid) gebildete polymere Gerüststruktur.

Die Vorrichtung 100 umfasst einen evakuierbaren Rezipienten 10 mit einer Einrichtung zum Erzeugen eines Hochfrequenzplasmas im Innenraum 15 des Rezipienten 10. Die Einrichtung zum Erzeugen eines Hochfrequenzplasmas ist im Schema der Figur 1 mittels zweier Elektroden 11 und 12 symbolisiert, jedoch nicht auf die Verwendung von Elektroden beschränkt. Es wird darauf hingewiesen, dass in Figur 1 im Hinblick auf eine klare und übersichtliche Darstellung nur diejenigen Komponenten dargestellt sind, die als erforderlich für das Verständnis der Erfindung erachtet werden. Komponenten, wie z. B. Pumpen zum Evakuieren des Rezipienten 10, die für den Betrieb der Vorrichtung zwar unerlässlich sind, für das Verständnis der Erfindung jedoch ohne Bedeutung, sind im Rahmen dieser Offenbarung trotz fehlender Darstellung als vorhanden unterstellt. Dem Innenraum 15 des Rezipienten 10 sind zumindest eine Vakuum- bzw. Niederdruckmesseinrichtung 13 und eine Beschichtungsauftragsmesseinrichtung 14, beispielsweise ein Schwingquarz, zugeordnet.

Die Beschichtungsvorrichtung 100 weist ferner eine Inertgasquelle 21 und eine oder mehrere Beschichtungsmaterialquellen 22 und 23 auf. Jede der Quellen bzw. Quellbehälter 21, 22 und 23 ist jeweils über eine der Leitungen 71, 72 und 73 so mit dem Rezipienten 10 verbunden, dass in den Quellen vorrätig gehaltene gasförmige bzw. in die Dampfphase überführte Stoffe in den Innenraum 15 des Rezipienten 10 geleitet werden können. In den Leitungen 71, 72 und 73 angeordnete Steuerventile 41, 42 und 43 ermöglichen eine Regelung des jeweiligen Gas- bzw. Dampfflusses in den Rezipienten 10. In dem dargestellten Ausführungsbeispiel können die Steuerventile alternativ auch zum Entlüften der Quellbehälter 21, 22 und 23 verwendet werden. Bei anderen Ausführungsformen werden hierfür eigene Ventile und gegebenenfalls auch separate Leitungen verwendet.

Die Vorrichtung 100 weist ferner eine Steuerung 80 auf, die z. B. mittels von Steuerleitungen 61, 62, 63, 64, 65 und Signalleitungen 66 und 67 zur gegebenenfalls geregelten Steuerung von Beschichtungsprozessen ausgebildet ist. Je nach Ausführungsform kann die Steuerung zur Ausführung vollautomatischer oder teilautomatischer Beschichtungsprozesse oder zur wahlweise voll- bzw. teilautomatischen Beschichtungssteuerung ausgebildet sein. Es sei in diesem Zusammenhang darauf hingewiesen, dass abweichend vom deutschen Sprachgebrauch in dieser Schrift nicht zwischen den Begriffen Steuern und Regeln unterschieden wird. Vielmehr werden beide Begriffe synonym verwendet, d.h. der Begriff Steuern kann ebenso eine Rückführung einer Regelgröße bzw. deren Messwerts umfassen, wie sich der Begriff Regeln auf eine einfache Steuerkette beziehen kann. Dies betrifft auch grammatikalische Abwandlungen dieser Begriffe. Eine geregelte (Teil-)Steuerung der Vorrichtung 100 kann z. B. unter Verwendung der Ausgangssignale von dem Innenraum 15 zugeordneten Sensoreinrichtungen realisiert sein. Beispielsweise können die Ventile 41, 42 und 43 unter Verwendung der Vakuum- bzw. Niederdruckmesseinrichtung 13 so gesteuert werden, dass im Innenraum 15 des Rezipienten 10 ein vorgegebener konstanter Gas- bzw. Dampfdruck mit ebenfalls vorgegebenen Partialdrücken aufrechterhalten wird. Ferner kann die Steuereinrichtung 80 dazu ausgebildet sein, das Aufwachsen einer Beschichtung mithilfe der Beschichtungsauftragsmesseinrichtung 14 zu überwachen und bei Erreichen der gewünschten Beschichtungsstärke zu beenden. Außerdem ist die Steuerung 80 in der Regel zur prozessablaufabhängigen Steuerung der Hochfrequenzeinrichtung 11 und 12 ausgebildet.

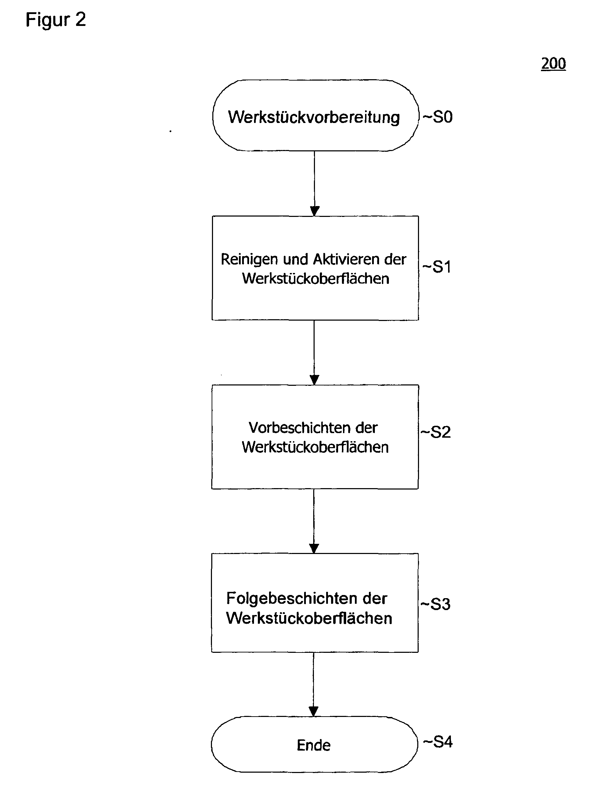

Das Flussdiagramm 200 der Figur 2 veranschaulicht die wesentlichen Schritte eines Verfahrens zum Hydrophilieren von Werkstückoberflächen durch Beschichten mit Polyacrylsäure. Vorzugsweise bilden polymere Biomaterialien die zur Herstellung der Werkstücke 90 bzw. deren Oberflächenbereichen verwendeten Werkstoffe, wobei unter dem Begriff "Biomaterial" alle Werkstoffe verstanden werden, die, z. B. im Rahmen von therapeutischen oder diagnostischen Maßnahmen, zum Kontakt mit biologischem Gewebe oder Körperflüssigkeiten vorgesehen sind.

Nach der Vorbereitung der Werkstücke 90 in Schritt S0, die gegebenenfalls ein Reinigen der Werkstücke und deren Anordnen im Rezipienten 10 sowie ein anschließendes Evakuieren des Rezipienten umfasst, werden die Werkstückoberflächen zunächst in Schritt S1 für eine -nachfolgende Beschichtung vorbereitet.

Hierzu wird der mit dem oder den Werkstücken beladene Rezipient 10 zunächst mithilfe von (in den Figuren nicht gezeigten) Pumpen evakuiert, vorzugsweise auf einen Druck von maximal 10⁻⁴ mbar (10 mPa). Nach Erreichen des gewünschten Vakuumdrucks wird das Rezipienteninnere 15 bei fortdauerndem Pumpen mit einem Inertgas, vorzugsweise Argon, geflutet, wobei der Inertgaszufluss so auf die Pumpleistung abgestimmt wird, dass sich im Innenraum 15 des Rezipienten 10 ein konstanter Gasdruck ausbildet. Das Inertgas 31 wird dem Rezipienten aus einer Inertgasquelle 21 zugeführt. Bei bevorzugten Ausführungsformen beträgt der so eingestellte Argongasdruck in etwa 25 mTorr (ca. 3,33Pa). Nach Erreichen eines stabilen Inertgasdrucks im Rezipienteninneren 15 wird der Plasmagenerator, beispielsweise ein Hochspannungsgenerator, eingeschaltet, wodurch ein die Werkstücke 90 umgebendes Inertgasplasma geschaffen wird. Das Plasma reinigt die Werkstückoberflächen durch Entfernen von daran adsorbierten Stoffen und resultiert ferner in einer Aktivierung der Werkstückoberflächen durch Bildung von Ionen und freien Radikalen, die den nachfolgenden Aufpolymerisierungsprozess begünstigen.

Die Reinigungs- und Aktivierungswirkung kann in diesem ersten Schritt S1 über die Frequenz des Gasplasmas, die in das Plasma eingespeiste Leistung, die Einwirkzeit des Plasmas und die Art des für das Plasma verwendeten Inertgases wie allgemein bekannt beeinflusst werden. Die für den jeweiligen Anwendungsfall geeigneten Einstellungen können in üblicher Weise vom Fachmann ermittelt werden. Als Inertgas wird bei dem hier vorgestellten Verfahren Argon bevorzugt, da es eine Aktivierung der Werkstoffoberflächen ohne Erzeugen neuer unerwünschter Verbindungen ermöglicht. Selbstverständlich können stattdessen auch andere Inertgase verwendet werden, wenn sie zu vergleichbaren Resultaten führen. Bei einer beispielhaften Ausführungsform des Verfahrens beträgt die Einwirkzeit des Argonplasmas in etwa eine Minute. Nach Ablauf der Einwirkzeit wird der Plasmagenerator ausgeschaltet und das Verfahren mit dem ersten Beschichtungsschritt S2 fortgeführt.

Abweichend vom bisher Dargestellten kann das zur Werkstückvorbereitung verwendete Plasma statt auf Basis reinen Argons auch auf Basis eines Gemisches von Inertgas und einer im nachfolgenden Vorbeschichtungsprozess verwendeten Reaktivkomponente erzeugt werden. Der Partialdruck der Reaktivkomponente sollte im Gasgemisch jedoch weniger als ein Zehntel des Partialdrucks des Inertgases betragen.

Beim Übergang von Schritt S1 zu Schritt S2 des Verfahrens wird der Inertgaszufluß ins Rezipienteninnere vorzugsweise aufrechterhalten und gegebenenfalls so geändert, dass er einen zur Durchführung von Schritt S2 geeigneten Wert aufweist. Zur Herstellung des Gasgemisches wird dem Inertgas im Rezipienten 10 ein aus biokompatiblen, polymerisierbaren carboxygruppenhaltigen Monomeren in der Dampfphase gebildetes erstes Schichtmaterialgas zugemischt.

Bei den carboxygruppenhaltigen Monomeren handelt es sich vorzugsweise um Acrylsäure oder einen Acrylsäurevorläufer, wie z. B. (Meth)Acrylsäureanhydrid. Der Partialdruck P_{esG} des ersten Schichtmaterialgases beträgt bei Ausführungsformen vorzugsweise mindestens ein Viertel und maximal das Doppelte des Partialdrucks PIG des Inertgases. Besonders bevorzugt ist das Partialdruckverhältnis P_{eSG}:P_{IG} aus einem Bereich von 1:1 bis 1:0,5 ausgewählt. Beispielsweise beträgt der Partialdruck von Argon bei Ausführungsformen des Verfahrens 30 mTorr (ca. 400 mPa) bei einem Gesamtdruck des Gasgemisches von 45 mTorr (ca. 600 mPa), woraus sich ein Wert von 2:1 bezüglich des Verhältnisses von Argonpartialdruck p_{Ar} zu erstem Schichtmaterialgaspartialdruck (Reaktivkomponentenpartialdruck) P_{esG} ergibt.

Als Reaktivkomponente zur Ausbildung des ersten Schichtmaterialgases wird bevorzugt (Meth)Acrylsäureanhydrid verwendet, das in einem der in Figur 1 veranschaulichten Behälter 22 oder 23 in die Dampfphase überführt und über die Leitung 72 oder 73 in den Innenraum 15 des Rezipienten 10 geleitet wird. Der Partialdruck des Schichtmaterialgases wird über dessen Zufluss eingestellt, der mithilfe der Ventile 42 oder 43 gesteuert wird. Statt (Meth)Acrylsäureanhydrid kann natürlich auch (Meth)Acrylsäure verwendet werden. (Meth)Acrylsäure bzw. (Meth)Acrylsäureanhydrid werden in den Quellbehältern 22 oder 23 in flüssiger Form, beispielsweise in einer Menge von 150 mL, bereitgestellt. Um ein Polymerisieren der Acrylsäure bzw. deren Vorläufermaterialien zu verhindern oder zu verzögern, können diese mit Cu(I)-chlorid versetzt werden. Ferner werden die Reaktivkomponentenbehälter 22 bzw. 23 nach dem Befüllen solange entlüftet, bis keine Blasen mehr in der Reaktivkomponentenflüssigkeit aufsteigen. Der Dampfdruck der Reaktivkomponenten ist bei den üblichen Raumtemperaturen von 22 bis 25 °C zur Bildung des ersten Schichtmaterialgases in der Regel ausreichend.

Nach Einstellen des gewünschten Gasgemisches und Gasgemischdrucks wird der eigentliche Vorbeschichtungsvorgang durch Starten des Plasmagenerators eingeleitet, wodurch im Plasma erzeugte angeregte Acrylsäuremonomere sich an der aktivierten Werkstückoberfläche anlagern und im weiteren Verlauf dort eine Polyacrylsäureschicht ausbilden. Diese plasmaunterstützte Vorbeschichtungsphase wird aufrechterhalten, bis eine vorgegebene Schichtdicke erreicht ist. Das Aufwachsen der Beschichtung wird dabei fortlaufend mithilfe der Beschichtungsauftragsmesseinrichtung 14 überwacht. Prinzipiell können Beschichtungen mit Dicken von bis zu 30.000 nm, respektive 30 µm abgeschieden werden, wobei ein jeweiliger Beschichtungsprozess beendet wird, sobald die Beschichtungsauftragsmesseinrichtung 14 das Erreichen der gewünschten Beschichtungsdicke innerhalb einer vorgegebenen Toleranz von z. B. 50 bis 400 Å (5 - 40 nm) anzeigt. Die Dicke der im Vorbeschichtungsprozess abzuscheidenden hydrophilen Beschichtung richtet sich nach dem jeweiligen Anwendungsfall und liegt bei Gerüstsubstanzen für Tissue Engineering in der Regel im Bereich von 30 bis 50 nm. Zum Hydrophilieren von Kontaktlinsen haben sich beispielsweise Vorbeschichtungen mit Dicken aus einem Bereich von etwa 5 bis 40 nm als geignet erwiesen. Je nach Anwendungsfall und damit auch der zu erzielenden Beschichtungsstärke, kann die Vorbeschichtungsphase zwischen 10 und 80 oder auch 120 Minuten dauern. Die Gaszufuhren werden während der Plasmabeschichtung vorzugsweise nicht verändert. Bei einer ersten Variante des Verfahrens wird der Vorbeschichtungsprozess durch Abschalten des Plasmagenerators beendet.

Der beschriebenen ersten Variante des Vorbeschichtungsschritts S2 schließt sich eine erste Variante des Folgebeschichtungsschritts S3 an, bei der nach dem Ausschalten des Plasmagenerators zunächst der Inertgaszufluss unterbrochen und die vorbeschichtete Werkstückoberfläche dem möglichst vollen Dampfdruck einer von wasserfreier Acrylsäure gebildeten Reaktivkomponente ausgesetzt wird. Der Dampfdruck der Reaktivkomponente sollte 5 Torr (ca. 667 Pa) nicht unterschreiten. Leichtes Kühlen oder Erwärmen der Reaktivkomponente im Quellbehälter 22 oder 23 kann dabei zur Druckeinstellung zweckmäßig sein. Das Einbringen der Reaktivkomponente in den Rezipienten 10 bei vollem Dampfdruck stellt Reaktivgas in großen Mengen bereit, das mit den an der vorbeschichteten Oberfläche vorhandenen reaktiven Zentren reagiert und eine vergleichsweise dicke Polyacrylsäureschicht (PAA-Schicht) aufbaut, die kristallin sein kann.

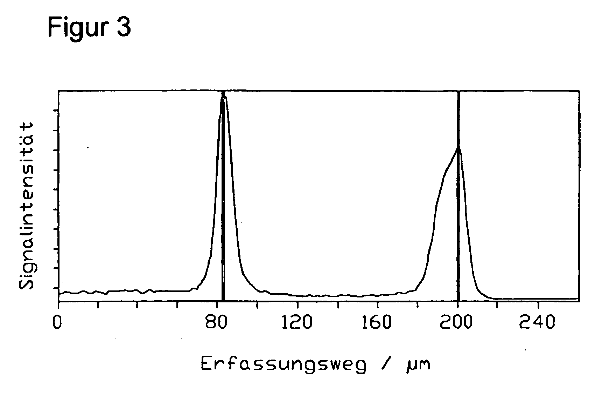

In Figur 3 ist ein Messdiagramm dargestellt, dem entnommen werden kann, dass eine wie oben beschrieben hergestellte PAA-Schicht eine Dicke von etwa 10 µm hat. Für die Messung wurde die hydrophile PAA-Schicht mit Rhodamin 6G als Fluoreszenzfarbstoff eingefärbt und die Fluoreszenz mittels konfokaler Mikroskopie dickenaufgelöst vermessen. Die hydrophile Schicht erstreckt sich, wie an dem rechten Bereich des Fluoreszenzsignalverlaufs deutlich ist, erkennbar in die Tiefe des Werkstücks. Die in Figur 3 vermessene Kontaktlinse hat an der Messstelle eine Dicke von 117,5 µm. Die Auflösung der Messung beträgt 0,6 µm. Aus den erhaltenen Daten kann von einer Beschichtungsstärke auf den Oberflächen von ca. 10±0,6 µm und einer Eindringtiefe pro Seite von ca. 15 bis 20±0,6 µm ausgegangen werden. In der beschriebenen Variante eignet sich das Verfahren daher besonders vorteilhaft zur Anwendung an Silicon-Kontaktlinsen, bei denen Hydrophilie der Oberfläche, Dauerhaftigkeit der Beschichtung sowie deren optische Eigenschaften gleichermaßen bedeutend sind.

Bei einer zweiten Variante des Verfahrens wird der Plasmagenerator am Ende des Vorbeschichtungsschrittes S2 nicht abgeschaltet und ist daher auch beim Übergang zur zweiten Variante des Folgebeschichtungsschrittes S3 noch in Betrieb. Bei dieser Variante wird die Argonzufuhr fast oder ganz gestoppt und die Zufuhr des Reaktivgases, d.h. der Acrylsäure, so weit reduziert, dass, bei aufrechterhaltener Hochfrequenzerzeugung und fortlaufendem Evakuieren des Rezipienten 10, ein Druckgleichgewicht im Bereich von weniger als 0,3 mTorr (ca. 40 mPa) eingestellt wird. In einer beispielhaften Ausführungsform wird der Druck auf einen Wert von weniger als 0,1 mTorr (ca. 13 mPa) eingestellt. Diese Folgebeschichtungsphase wird für 5 bis 15 Minuten aufrechterhalten und resultiert bei porösen resorbierbaren Gerüstsubstanzen für Tissue Engineering in Werkstückoberflächen, die besonders niedrige Kontaktwinkel für Wasser und hervorragende Zellanhaftungsraten von z.B. über 90% oder über 95% aufweisen. Die beschriebene zweite Variante des Verfahrens ist daher besonders zur Herstellung beschichteter Gerüstsubstanzen geeignet, die zur Infiltration von Zellen im Rahmen eines Tissue Engineerings dienen sollen.

Bei Beendigung des Verfahrens in Schritt S4 können die beschichteten Werkstücke 90 aus dem Rezipienten entnommen und gegebenefalls einer Qualitätskontrolle unterzogen werden.

Das beschriebene Verfahren ermöglicht eine dauerhafte Hydrophilierung polymerer Biomaterialoberflächen, die eine ausgezeichnete Benetzung mit Wasser und damit eine hohe Bioverträglichkeit möglich machen.

## Patentansprüche

1. Verfahren zum Hydrophilieren von Oberflächen polymerer Werkstücke, wobei das Verfahren Schritte umfasst zum
(a) Vorbehandeln der Werkstückoberflächen in einem auf Grundlage eines Inertgases gebildeten Hochfrequenzgasplasmas zum Reinigen und Aktivieren der Werkstückoberflächen,
(b) Vorbeschichten der vorbehandelten Werkstückoberflächen mit Polyacrylsäure unter Verwendung eines aus einer Gasmischung erzeugten Hochfrequenzgasplasmas, wobei sich die Gasmischung aus einem Inertgas und einem aus biokompatiblen, polymerisierbaren carboxygruppenhaltigen Monomeren gebildeten ersten Gas zusammensetzt, und
**dadurch gekennzeichnet ist, dass** es unmittelbar anschließend ferner Schritte umfasst zum
(cb)Drosseln der Inertgaszufuhr, Zuführen eines im Wesentlichen Acrylsäuremonomere enthaltenden zweiten Gases und
(c) Folgebeschichten der vorbeschichteten Werkstückoberflächen unter Verwendung des zweiten Gases.

2. Verfahren nach Anspruch 1, worin die das erste Gas bildenden biokompatiblen, polymerisierbaren carboxygruppenhaltigen Monomere ausgewählt sind unter (Meth)Acrylsäure und (Meth)Acrylsäureanhydrid.

3. Verfahren nach Anspruch 1 oder 2, worin das in Schritt (a) zur Ausbildung des Hochfrequenzplasmas verwendete Gas das erste Gas in einer Menge enthält, die einem Partialdruck von weniger als einem Zehntel des Partialdrucks des Inertgases entspricht.

4. Verfahren nach Anspruch 1, 2 oder 3, worin bei einer in Schritt (b) verwendeten Gasmischung der Partialdruck des ersten Gases mindestens ein Viertel und maximal das Doppelte des Partialdrucks des Inertgases beträgt.

5. Verfahren nach einem der vorhergehenden Ansprüche, worin der Partialdruck des Inertgases im in Schritt (c) verwendeten zweiten Gas weniger als ein Zehntel des Partialdrucks des von Acrylsäuremonomeren gebildeten Gases beträgt.

6. Verfahren nach einem der vorhergehenden Ansprüche, worin Argon das Inertgas bildet.

7. Verfahren nach einem der vorhergehenden Ansprüche, worin die in Schritt (b) aufgebrachte Beschichtung mithilfe einer Schichtdickenkontrolleinrichtung überwacht und bei Erreichen eines aus dem Bereich von 50 bis 400 Å (5 - 40 nm) ausgewählten Schichtdickenwerts beendet wird.

8. Verfahren nach einem der vorhergehenden Ansprüche, worin der Druck des Inertgases für das Hochfrequenzplasma in Schritt (a) in 15 bis 60 mTorr (ca. 2 bis 8 Pa) und der Druck des ersten Gases für das Hochfrequenzplasma in Schritt (b) 30 bis 90 mTorr (ca. 4 bis 12 Pa) beträgt.

9. Verfahren nach einem der vorhergehenden Ansprüche, wobei der Druck des zweiten Gases in Schritt (c) weniger als 0,3 mTorr (ca. 40 mPa) beträgt.

10. Verfahren nach einem der vorhergehenden Ansprüche, das ferner einen Schritt (bc) aufweist, der unmittelbar an Schritt (b) oder an Schritt (cb) anschließend ein Ausschalten des Hochfrequenzplasmas, ein Unterbrechen der Inertgaszufuhr und ein Zuführen des zweiten Gases umfasst, wobei der Druck des zweiten Gases in Schritt (c) zwischen 1,5 und 6 Torr (ca. 0,13 bis 0,8 kPa) beträgt.

11. Verfahren nach einem der vorhergehenden Ansprüche, das ferner einen an Schritt (c) anschließenden weiteren Schritt (d) zum Entfernen wasserlöslicher Bestandteile aus der Hydrophilierungsschicht mittels Spülen des beschichteten Werkstücks in einem hydrophilen Lösungsmittel wie isotonischer Kochsalzlösung oder demineralisiertem Wasser umfasst.

12. Verfahren nach einem der vorhergehenden Ansprüche, worin das Werkstück zumindest an seiner Oberfläche überwiegend oder im Wesentlichen aus einem Silicon, insbesondere Poly(dimethylsiloxan), einem Siliconhydrogel oder einem porösen bioresorbierbaren Polymer wie PLA oder PLGA gebildet ist.

13. Polymeres Werkstück mit einer hydrophilierenden Oberflächenbeschichtung aus Polyacrylsäure erhältlich nach einem Verfahren gemäß einem der Ansprüche 1 bis 12, wobei der Benetzungswinkel von Wasser an der mit Polyacrylsäure beschichteten Werkstückoberfläche einen Wert aus dem Bereich von 2 bis unter 10 Grad aufweist.

14. Polymeres Werkstück nach Anspruch 13, wobei es sich um eine Silikon-Kontaktlinse handelt und die PAA-Schicht eine durchschnittliche Dicke von 5-40 µm aufweist.

15. Polymeres Werkstück nach Anspruch 13, wobei es sich um eine poröse Matrix aus Poly(α-hydroxycarbonsäuren) handelt und die PAA-Schicht eine durchschnittliche Dicke von 5 bis 40 nm aufweist.

## Claims

1. A process for hydrophilizing surfaces of polymeric workpieces, the process comprising steps for
(a) pre-treating the workpiece surfaces in a high frequency gas plasma generated on the basis of an inert gas, for cleaning and activating the workpiece surfaces,
(b) pre-coating the pre-treated workpiece surfaces with polyacrylic acid using a high frequency gas plasma generated using a gas mixture, wherein the gas mixture is composed of an inert gas and a first gas constituted of biocompatible, polymerizable carboxy group-containing monomers, and
**characterized in that** it comprises immediately subsequent steps for
(cb)reducing the inert gas supply, supplying a second gas substantially containing acrylic acid monomers and
(c) follow-up coating the pre-coated workpiece surfaces using the second gas.

2. The process of claim 1, wherein the biocompatible, polymerizable carboxy group-containing monomers constituting the first gas are selected from among (meth)acrylic acid and (meth)acrylic acid anhydride.

3. The process of claim 1 or 2, wherein the gas used in step (a) for forming the high frequency plasma contains the first gas in an amount corresponding to a partial pressure of less than one tenth of the partial pressure of the inert gas.

4. The process of claim 1, 2 or 3, wherein in a gas mixture used in step (b), the partial pressure of the first gas is at least one quarter and maximally twice the partial pressure of the inert gas.

5. The process of one of the preceding claims, wherein the partial pressure of the inert gas in the second gas used in step (c) is less than one tenth of the partial pressure of the second gas constituted of acrylic acid monomers.

6. The process of one of the preceding claims, wherein Argon constitutes the inert gas.

7. The process of one of the preceding claims, wherein the coating applied in step (b) is monitored by means of a coating thickness control apparatus and is terminated on reaching a coating thickness value selected from the range of 50 to 400 Å.

8. The process of one of the preceding claims, wherein the pressure of the inert gas for the high frequency plasma in step (a) is 15 to 60 mTorr (ca. 2 to 8 Pa) and the pressure of the first gas for the high frequency plasma in step (b) is 30 to 90 mTorr (ca. 4 to 12 Pa).

9. The process of one of the preceding claims, wherein the pressure of the second gas in step (c) is less than 0.3 mTorr (ca. 40 mPa).

10. The process of one of the preceding claims, further comprising a step (bc), immediately subsequent to step (b) or step (cb) comprising switching off the high frequency plasma, interrupting the inert gas supply, and supplying the second gas, wherein the pressure of the second gas in step (c) is between 1.5 and 6 Torr (ca. 0.13 to 0.8 kPa).

11. The process of one of the preceding claims, further comprising a step (d), subsequent to step (c), for removing water soluble constituents from the hydrophilizing layer by rinsing the coated workpiece in a hydrophilic solvent such as isotonic saline solution or demineralized water.

12. The process of one of the preceding claims, wherein the workpiece is formed, at least at its surface, mainly or substantially from a silicone, in particular poly (dimethylsiloxane), a silicone hydrogel, or a porous bioresorbable polymer such as PLA or PLGA.

13. A polymeric workpiece having a hydrophilizing surface coating of polyacrylic acid, obtainable by the process of one of claims 1 to 12, wherein the contact angle of water at the workpiece surface coated with polyacrylic acid has a value in the range of 2 degrees to less than 10 degrees.

14. The polymeric workpiece of claim 13, wherein it is a silicone contact lens and the PAA layer has an average thickness of 5-40 µm.

15. The polymeric workpiece of claim 13, wherein it is a porous matrix of poly(α-hydroxycarboxylic acids) and the PAA layer has an average thickness of 5 to 40 nm.

## Revendications

1. Procédé d'hydrophilisation de surfaces de pièces usinées en polymère, le procédé comprenant des étapes de
(a) prétraitement des surfaces des pièces usinées dans un plasma gazeux à haute fréquence formé sur la base d'un gaz inerte pour nettoyer et activer les surfaces de pièces usinées,
(b) prérevêtement des surfaces de pièces usinées prétraitées avec de l'acide polyacrylique en utilisant un plasma gazeux à haute fréquence produit à partir d'un mélange gazeux, le mélange gazeux étant composé d'un gaz inerte et d'un monomère contenant des groupes carboxyle biocompatibles polymérisables, et
**caractérisé en ce qu'**il comprend immédiatement après, d'autres étapes de
(cb) réduction de l'apport de gaz inerte, apport d'un deuxième gaz contenant essentiellement des monomères d'acide acrylique et
(c) revêtement consécutif des surfaces de pièces usinées pré-revêtues en utilisant le deuxième gaz.

2. Procédé selon la revendication 1, dans lequel les monomères contenant des groupes carboxyle biocompatibles polymérisables, formant le premier gaz, sont choisis parmi l'acide (méth)acrylique et l'anhydride d'acide (méth)acrylique.

3. Procédé selon la revendication 1 ou 2, dans lequel le gaz utilisé dans l'étape (a) pour former le plasma à haute fréquence contient le premier gaz en une quantité qui correspond à une pression partielle de moins d'un dixième de la pression partielle du gaz inerte.

4. Procédé selon la revendication 1, 2 ou 3, dans lequel, pour un mélange gazeux utilisé à l'étape (b), la pression partielle du premier gaz est d'au moins un quart et au maximum le double de la pression partielle du gaz inerte.

5. Procédé selon l'une des revendications précédentes, dans lequel la pression partielle du gaz inerte dans le deuxième gaz utilisé à l'étape (c) est inférieure à un dixième de la pression partielle du gaz formé par des monomères d'acide acrylique.

6. Procédé selon l'une des revendications précédentes, dans lequel l'argon constitue le gaz inerte.

7. Procédé selon l'une des revendications précédentes, dans lequel le revêtement appliqué à l'étape (b) est surveillé à l'aide d'un dispositif de contrôle de l'épaisseur de couche et est terminé lorsque l'on obtient une valeur d'épaisseur de couche choisie dans la plage de 50 à 400 Å (5 à 40 µm).

8. Procédé selon l'une des revendications précédentes, dans lequel la pression de gaz inerte pour le plasma à haute fréquence à l'étape (a) est de 15 à 60 mtorrs (environ 2 à 8 Pa) et la pression du premier gaz pour le plasma à haute fréquence à l'étape (b) est de 30 à 90 mtorrs (environ 4 à 12 Pa).

9. Procédé selon l'une des revendications précédentes, la pression du deuxième gaz à l'étape (c) étant inférieure à 0,3 mtorrs (environ 40 mPa).

10. Procédé selon l'une des revendications précédentes, qui présente en outre une étape (bc) qui comprend immédiatement après l'étape (b) ou l'étape (cb), un arrêt du plasma à haute fréquence, une interruption de l'apport de gaz inerte et un apport du deuxième gaz, la pression du deuxième gaz à l'étape (c) se situant entre 1,5 et 6 torrs (environ 0,13 à 0,8 kPa).

11. Procédé selon l'une des revendications précédentes, qui comprend en outre une autre étape (d) après l'étape (c) pour éliminer les composants solubles dans l'eau de la couche d'hydrophilisation par rinçage de la pièce usinée revêtue dans un solvant hydrophile tel qu'une solution saline isotonique ou de l'eau déminéralisée.

12. Procédé selon l'une des revendications précédentes, dans lequel la pièce usinée est constituée au moins sur sa surface, principalement ou essentiellement, de silicone, en particulier de poly(diméthylsiloxane), d'un hydrogel de silicone ou d'un polymère poreux biorésorbable tel que le PLA ou le PLGA.

13. Pièce usinée en polymère, comportant un revêtement de surface hydrophilisant d'acide polyacrylique, pouvant être obtenue selon un procédé selon l'une des revendications 1 à 12, l'angle de mouillage de l'eau à la surface de la pièce usinée revêtue avec de l'acide polyacrylique présentant une valeur dans la plage de 2 à 10 degrés.

14. Pièce usinée en polymère selon la revendication 13, celle-ci étant une lentille de contact en silicone et la couche de PAA présentant une épaisseur moyenne de 5 à 40 µm.

15. Pièce usinée en polymère selon la revendication 13, celle-ci étant une matrice poreuse d'acides poly(α-hydroxycarboxylique) et la couche de PAA présentant une épaisseur moyenne de 5 à 40 nm.
